# EUROPEAN PATENT SPECIFICATION

(11) **EP 1 534 271 B1**
(45) Date of publication and mention of the grant of the patent: **23.08.2006**
(21) Application number: 03765081.9
(22) Date of filing: 22.07.2003
(51) Int. Cl.: A61K 31/403, A61P 35/00, A61P 9/00

(54) **ANTITUMOR COMPOUND AND THERAPEUTIC USES THEREOF**
ANTITUMORVERBINDUNG UND IHRE THERAPEUTISCHEN VERWENDUNGEN
COMPOSE ANTITUMORAL ET SES UTILISATIONS THERAPEUTIQUES

(30) Priority: 23.07.2002 IT MI20021620
(43) Date of publication of application: 01.06.2005
(73) Proprietor: Cell Therapeutics Europe S.R.L., 20091 Bresso (IT); ISTITUTO NAZIONALE PER LO STUDIO E LA CURA DEI TUMORI, I-20133 Milano (IT)
(72) Inventor: LANZI, Cinzia, I-20091 Bresso (IT); CASSINELLI, Giuliana, I-20091 Bresso (IT); CUCCURU, Giuditta, I-20091 Bresso (IT); PIEROTTI, Marco, Alessandro, I-20091 Bresso (IT); ZUNINO, Franco, I-20091 Bresso (IT); MENTA, Ernesto, I-20091 Bresso (IT)
(74) Representative: Minoja, Fabrizio
(86) International application number: PCT/EP2003/007963
(87) International publication number: WO 2004/009083

(56) References cited:
- US-A- 6 130 238
- US-A- 6 133 305
- US-A- 6 147 106
- US-B1- 6 268 391
- CINZIA LANZI AND ALL.: "Inhibition of transforming activity of the ret/ptc1 oncoprotein by a 2-indolinone derivative" INT.J.CANCER, vol. 85, 2000, pages 384-390, XP002257784 cited in the application
- SUAREZ H G: "Molecular basis of epithelial thyroid tumorigenesis" COMPTES RENDUS DES SEANCES DE L'ACADEMIE DES SCIENCES. SERIE III: SCIENCES DE LA VIE, ELSEVIER, AMSTERDAM, NL, vol. 323, no. 6, June 2000 (2000-06), pages 519-528, XP004330637 ISSN: 0764-4469
- SARASIN A ET AL: "Mechanisms of mutagenesis in mammalian cells. Application to human thyroid tumours" COMPTES RENDUS DES SEANCES DE L'ACADEMIE DES SCIENCES. SERIE III: SCIENCES DE LA VIE, ELSEVIER, AMSTERDAM, NL, vol. 322, no. 2-3, February 1999 (1999-02), pages 143-149, XP004270260 ISSN: 0764-4469
- HANKE J.H. ET AL: 'Discovery of a Novel, Potent, and Src Family-selective Tyrosine Kinase Inhibitor' THE JOURNAL OF BIOLOGICAL CHEMISTRY vol. 271, no. 2, 1996, pages 695 - 701
- LYDON N.B. ET AL: 'Lessons learned from the development of imatinib' LEUKEMIA RESEARCH vol. 28S1, 2004, pages S29 - S38
- RYAN A.J. ET AL: 'ZD6474 - a novel inhibitor of VEGFR and EGFR tyrosine kinase activity' BRITISH JOURNAL OF CANCER vol. 92, no. 1, 2005, pages S6 - S13
- TATTON L. ET AL: 'The Src-selective Kinase Inhibitor PP1 Also Inhibits Kit and Bcr-Abl Tyrosine Kinases' THE JOURNAL OF BIOLGICAL CHEMISTRY vol. 278, no. 7, 2003, pages 4847 - 4853
- WALTENBERGER J. ET AL: 'A Dual Inhibitor of Platelet-Derived Growth Factor beta-Receptor and Src Kinase Activity Potently Interferes With Motogenic and Mitogenic Responses to PDGF in Vascular Smooth Muscle Cells' CIRCULATION RESEARCH vol. 85, 1999, pages 12 - 22

## Description

The present invention regards the use of the compound (E)-1,3-dihydro-5,6-dimethoxy-3-[(4-hydroxyphenyl)methylene]-2H-indol-2-one in the treatment of tumors involving Met, PDGF-R, FGF-R1, FGF-R3 and Kit tyrosine kinases, or Ret oncoproteins.

### BACKGROUND ART

RET/PTC oncogenes are involved in the transforming processes of human papillary thyroid tumors and originate from the rearrangement of the tyrosine kinase domain of proto-RET with different donor genes. The products of such gene rearrangements show ligand-independeut tyrosine-kinase activity and are localized in the cytoplasm. Ret/ptc1 is the product of the RET/PTC1 oncogene, which originates from the rearrangement of the proto-RET tyrosine kinase domain with the H4/D10S170 gene.

Int. J. Cancer 85, 384-390 (2000) reports the tyrosine kinase activity inhibition of Ret/ptc1 oncoprotein by arylidene 2-indolinone compounds. The 1,3-dihydro-5,6-dimethoxy-3-[(4-hydroxyphenyl)methylene]-2H-indol-2-one derivative (hereafter "Cpd 1") is indicated as particularly effective in reverting the morphologic phenotype of NIH3T3 cells transformed with the RET/PTC1 oncogene. In the same paper, tyrosine kinase inhibition by arylidene 2-indolinone compounds is proposed for the study of Ret signaling and for controlling cell proliferation in Ret- and Ret/ptcs-associated diseases.

US 6268391 discloses tyrosine kinase inhibitors structurally related to Cpd 1.

### DESCRIPTION OF THE INVENTION

Cpd 1 has now been found to effectively inhibit tyrosine kinases, other than Ret/ptcl, that play a central role in tumor onset, progression and spreading to distant organs. Specifically, Cpd 1 has been shown to completely inhibit the autophosphorylation of Ret/MEN2A (mutations C634R and C634W), Ret/MEN2B (mutM918T), Met, PDGF-R, FGF-R1, FGF-R3 and Kit (c-Kit and mutΔ559) tyrosine kinases, to reduce their expression (down-regulation) and to revert the phenotype of cells thereby transformed.

Object of the invention is therefore the use of the compound (E)-1,3-dihydro-5,6-dimethoxy-3-[(4-hydroxyphenyl)methylene]-2H-indol-2-one, or of its salts with pharmaceutically acceptable bases, for the treatment of tumors involving at least one of Met, PDGF-R, FGF-R1, FGF-R3 and Kit tyrosine kinases, including Ret receptors carrying MEN2-associated mutations, in the initial stages of cell transformation or in the following stages of tumor proliferation and dissemination.

The invention also concerns the use of any stereoisomer or tautomeric form of Cpd.1.

Inhibition of deregulated, constitutively active, Ret receptors is useful in the treatment of sporadic medullary thyroid carcinomas (MTC) and MEN2-associated diseases including MTC, pheochromocytoma, parathyroid hyperplasia, and enteric ganglioneuromas.

Met inhibition is useful to antagonize the invasive/metastatic phenotype of tumors of epithelial origin. With respect to Met-activating alterations, Cpd 1 may also have a specific indication in the therapy of kidney tumors.

Kit inhibition is useful in the treatment of gastrointestinal stromal tumors, small cell lung carcinomas, seminomas and hematological malignancies such as mastocytosis and acute myelogenous leukemia.

The uncontrolled activation of PDGF-R and its involvement in autocrine loops support the therapeutic use of Cpd 1 in tumors unresponsive to conventional therapies, such as glioma and dermatofibrosarcoma protuberans. In addition, PDGF-R is involved in tumor angiogenesis and vascular development thus supporting the use of Cpd1 for the control of neoangiogenesis in solid tumors.

Cpd 1 can be used for the treatment of melanomas and gliomas expressing high levels of FGF-R1 and of the respective bFGF ligand eventually involved in autocrine loops. Since this receptor has an important role in angiogenic processes, its inhibition by means of Cpd 1 is useful for the control of tumor vascularization.

FGF-R3 inhibition is useful in the treatment of multiple myeloma, bladder and cervix carcinomas.

For use in therapy, RPI-1 and its salts can be formulated with pharmaceutically acceptable vehicles and excipients. The phenol function of Cpd 1 can be salified by treatment with suitable organic or inorganic bases. The pharmaceutical compositions can be administered by the oral, parenteral, sublingual or transdermic routes, preferably in the form of tablets, capsules, granules, powders, syrups, solutions, suspensions, suppositories, controlled release forms.

The compositions can be prepared with conventional techniques, using ingredients known in the art. The quantity of active principle can be varied depending on the severity of the disease, age of the patient, type and route of administration, but in general an amount of 0.1 to 1000 mg/Kg, preferably from 5 to 300 mg/Kg, more preferably from 20 to 200 mg/Kg, in single or multiple doses one or more times a day, is used.

### DETAILED DESCRIPTION OF THE INVENTION

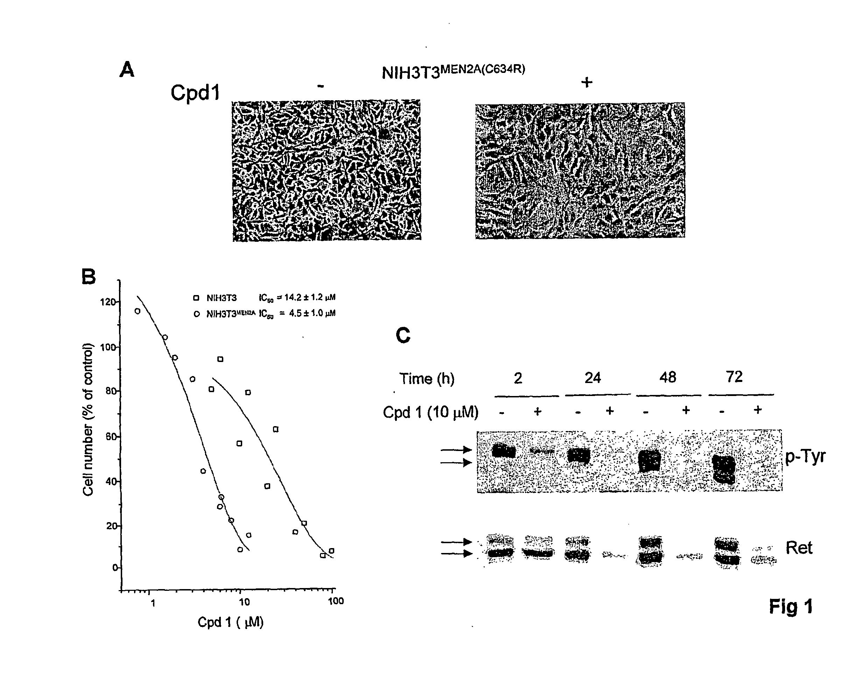

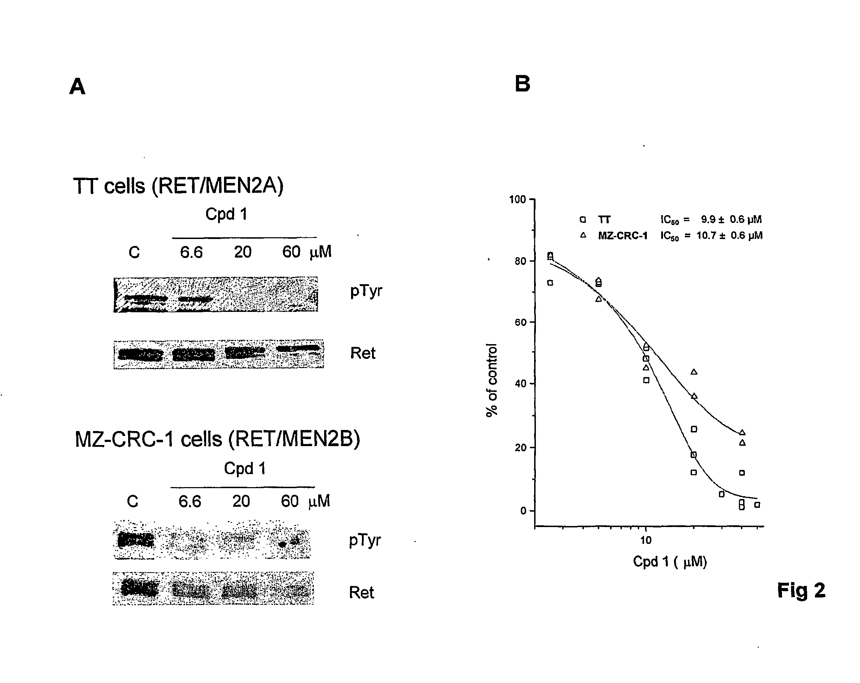

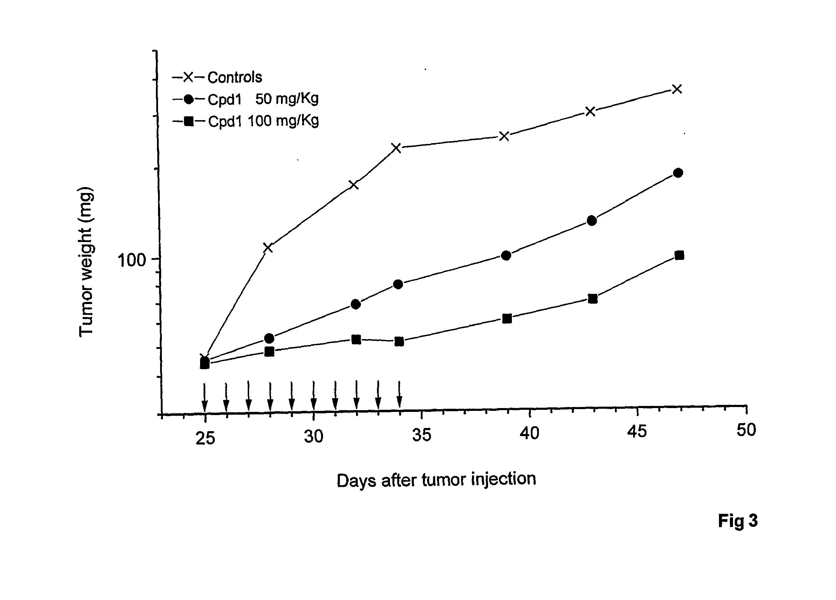

Ret oncoproteins carrying aminoacid substitutions which cause constitutive tyrosine kinase activity are involved in sporadic MTC and in the inherited type 2 Multiple Endocrine Neoplasia syndromes (MEN2A, MEN2B and Familial MTC), all characterized by the occurrence of MTC (Jhiang S.M. et al. Oncogene 19, 5590, 2000). Whereas in sporadic MTC RET mutations are somatic, in MEN2 patients RET mutations are present at the germline level. These mutations cause constitutive activation of the receptor without modifying its localization at the cell membrane. The Ret oncoproteins used in this study involve Cys634 (indicated as Ret/MEN2A^{C634R} and Ret/MEN2A^{C634W}) or Met918 (indicated as Ret/MEN2B^{M918T}), and represent the most frequently expressed Ret oncoproteins in MEN2A and MEN2B, respectively. The inhibitory effect of Cpd 1 has been demonstrated in murine cells transfected with the RET/MEN2A(C634R) gene (NIH3T3^{MEN2A(C634R)} cells) and in the human medullary thyroid carcinoma cell lines TT and MZ-CRC-1, respectively characterized by the expression of Ret/MEN2A(C634W) and Ret/MEN2B(M918T) (Figures 1, 2). A reduction of the oncoprotein tyrosine-phosphorylation and expression is observed in these cell lines (Figures 1C and 2A). The inhibition of Ret/MEN2A and Ret/MEN2B receptor autophosphorylation by Cpd 1 is associated with an antiproliferative effect (Figures 1B and 2B). NIH3T3^{MEN2A(C634R)} transfectants reverted their transformed phenotype following exposure to Cpd1 (Fig. 1A). A significant dose-dependent antitumor activity has been observed in nude mice xenografted with the TT tumor: after oral administration of a daily dose of 50-100 mg/Kg (twice a day), Cpd 1 treatment reached 80% tumor weight inhibition (TWI) without inducing toxicity (Fig. 3). The demonstrated pharmacological and biochemical efficacy of Cpd 1 in controlling the proliferation of MTC cells is particularly important considering the aggressiveness of such tumors and the inefficacy of conventional therapies.

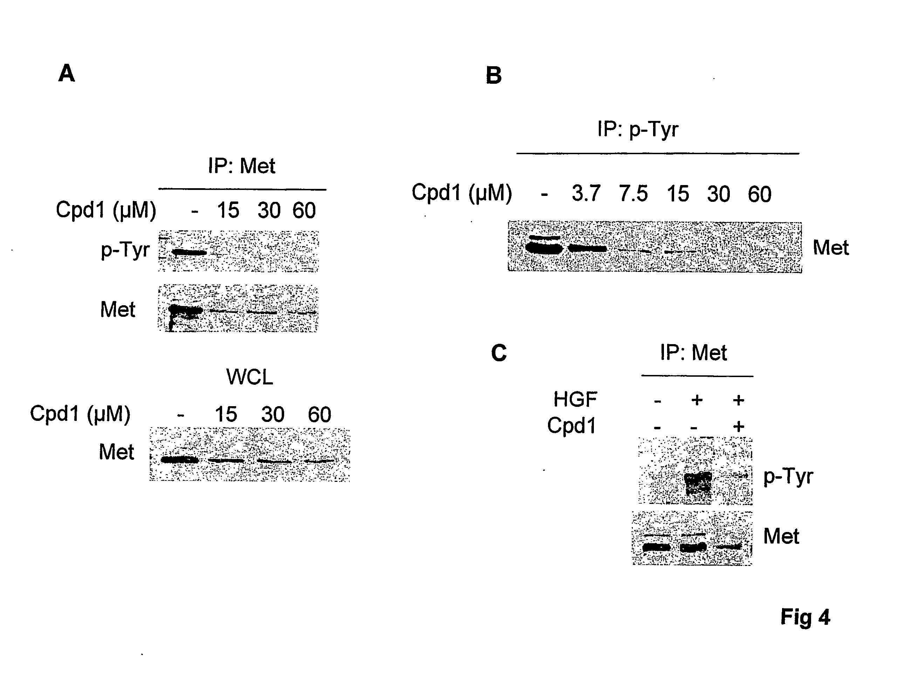

Met, the hepatocyte growth factor receptor, is a protein tyrosine kinase involved in the invasive process characteristic of tumor progression and metastatic growth (Maulik G. et al. Cytok. Growth Factor Rev. 13, 41, 2000). Alterations such as mutations, overexpression or the involvement in autocrine loops are the cause of uncontrolled constitutive activation of the kinase. The uncontrolled kinase activity of Met is involved in the invasiveness of many tumors of epithelial origin. In papillary thyroid carcinomas Met is frequently overexpressed. The results illustrated in Fig. 4 show a dose-dependent inhibition of Met autophosphorylation in the papillary thyroid carcinoma cell line TPC-1 treated with Cpd 1. Met protein levels are also reduced in treated cells.

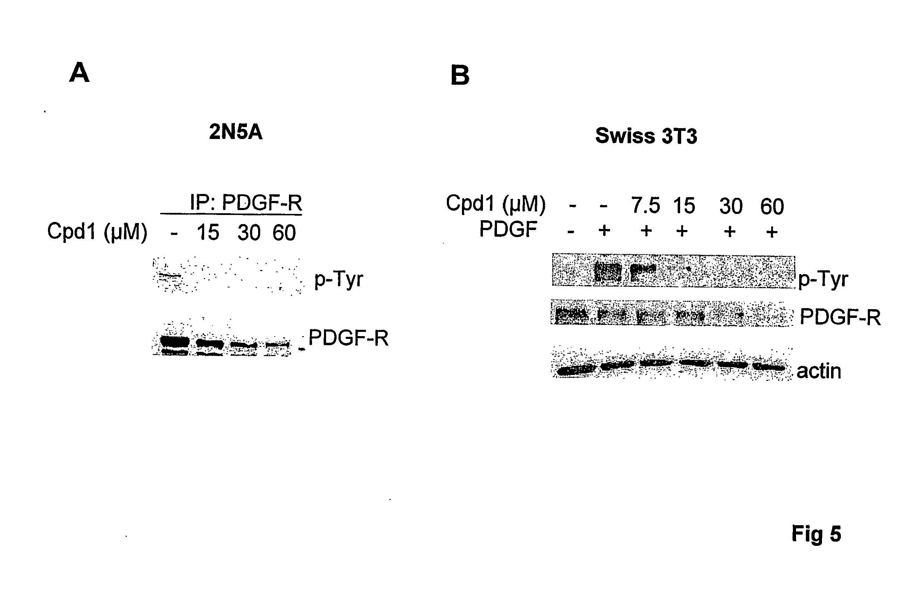

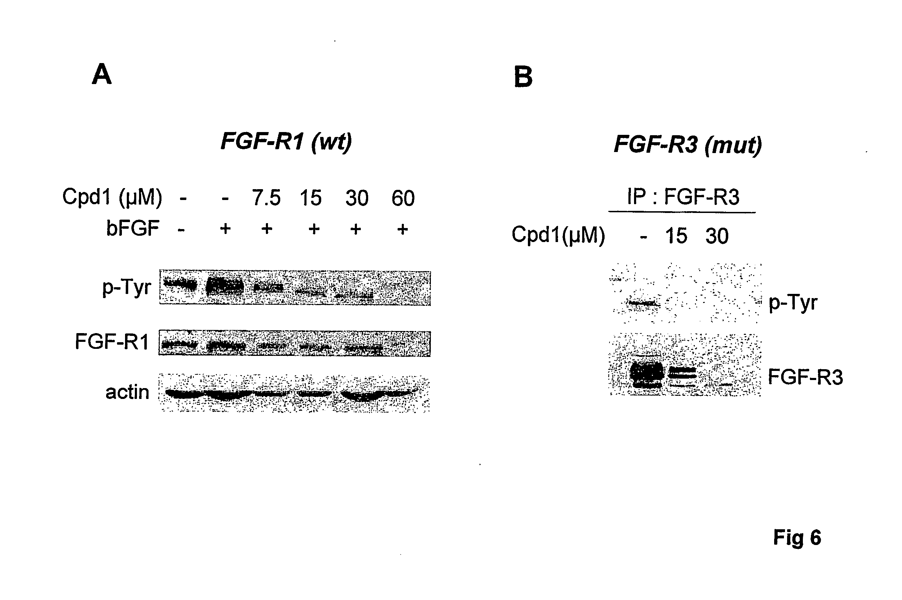

Other receptor tyrosine kinases, such as PDGF-R (Rosenkranz S. and Kazlauskas A. Growth Factors 16, 201, 1999) and FGF-R1 (Powers C.J. et al. Endocr. Rel. Cancer 7, 165, 2000), which are involved either in autocrine loops or in neoangiogenic processes, have an important role in cancer growth. A deregulated activation of these receptors is observed in tumors unresponsive to conventional therapies, such as gliomas and melanomas. The results illustrated in Figures. 5 and 6 show a dose-dependent inhibition by Cpd 1 of receptor autophosphorylation induced by autocrine stimulation (Fig. 5A) or by exogenous ligand (Figures 5B and 6A). These effects are associated with a reduced receptor expression. Concentrations of Cpd 1 higher than 15 µM cause full inhibition of PDGF-R phosphorylation.

Activating mutations of the FGF-R3 receptor tyrosine kinase such as chromosomal traslocations or point mutations produce deregulated, constitutively active, FGF-R3 receptors which have been involved in multiple mieloma and in bladder and cervix carcinomas (Powers C.J. et al. Endocr. Rel. Cancer 7, 165, 2000). The ability of Cpd 1 to down regulate both tyrosine autophosphorylation and expression of FGF-R3 (mutY373C) exogenously expressed in NIH3T3 transfectants is illustrated in Fig. 6B.

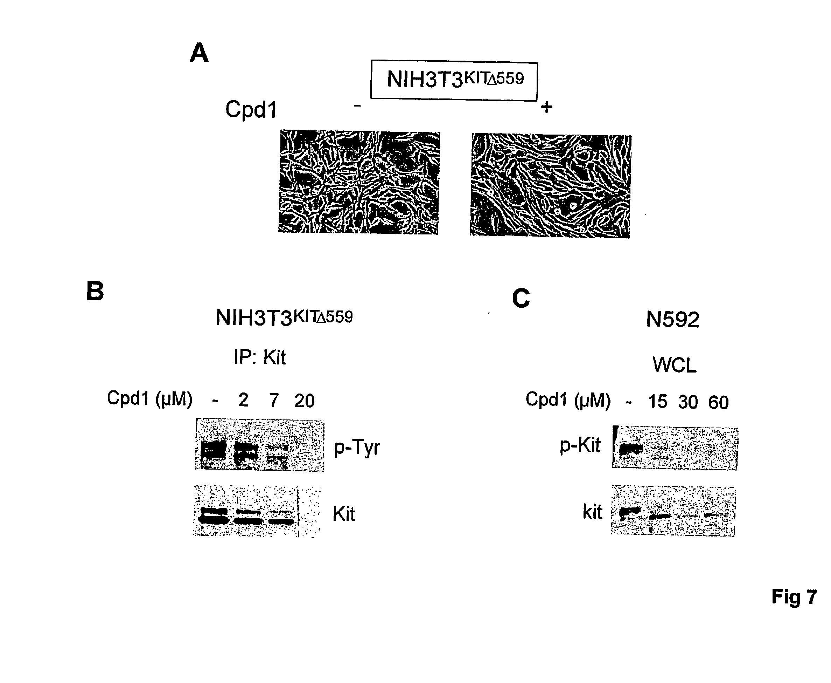

Kit tyrosine kinase is constitutively activated as a consequence of mutations or of its involvement in autocrine loops in different tumors such as small cell lung cancers, gastro-intestinal stromal tumors, seminomas and leukemias (Heinrich M.C. et al. J. Clin. Oncol. 20, 1692, 2002). As shown in Fig. 7, Cpd 1 inhibits the constitutive autophosphorylation and expression of the Kit (Δ559) mutant exogenously expressed in NIH3T3 cells (Fig. 7B). Such inhibition is associated with reversion of the transformed morphologic phenotype of the transfected cells (Fig. 7A). In addition, Fig. 7C shows the dose-dependent inhibition by Cpd 1 of c-Kit activated through autocrine loop in the small cell lung carcinoma cell line N592.

As used herein, the term "tumor" is intended to encompass the abnormal cell proliferation of malignant or non-malignant cells of various tissues and/or organs such as muscle, bone or connective tissue, the skin, brain, lungs, sex organs, the lymphatic or renal systems, mammary or blood cells, liver, the digestive system, pancreas and thyroid or adrenal glands. The abnormal cell proliferation can include tumors of the ovary, breast, brain, prostate, colon, liver, lung, ovary, uterus, cervix, pancreas, gastrointestinal tract, head, neck, nasopharynx, skin, bladder, stomach, kidney or testicles, Kaposi's sarcoma, cholangiocarcinoma, choriocarcinoma, neuroblastoma, Wilms' tumor, Hodgkin's disease, melanoma, multiple myeloma, chronic lymphocytic leukemia and acute or chronic granulocytic lymphoma.

The compounds according to the present invention can be administered alone or in combination with other anti-tumor or anti-cancer agents including adriamycin, daunomycin, methotrexate, vincristin, 6-mercaptopurine, cytosine arabinoside, cyclophosphamide, 5-FU, hexamethylmelamine, carboplatin, cisplatin, idarubycin, paclitaxel, docetaxel, topotecan, irinotecam, gemcitabine, L_PAM, BCNU and VP-16. The compounds according to the present invention can also be included in a kit for the treatment of tumors. The kit can include additional anti-cancer or anti-tumor agents.

The following Figures illustrate the invention in greater detail.

### Description of the Figures

**Figure 1.** Effects of Cpd 1 on NIH3T3^{MEN2A(C634R)} transfectants expressing exogenous RET/MEN2A(C634R). A) Reversion of the transformed morphologic phenotype of NIH3T3^{MEN2A(C634R)} cells. Cells were exposed to 6 µM Cpd1 for 24h and then photographed under a phase-contrast microscope (original magnification X 100). **B**) Antiproliferative effect. NIH3T3^{MEN2A(C634R)} cells and the parental NIH3T3 cells were treated with increasing concentrations of the drug for 72h and then counted with a Coulter Counter. Dose-response curves, from which the reported IC₅₀ values were calculated, showed the higher sensitivity to the drug of the Ret oncoprotein-positive cell line. C) Inhibition of Ret/MEN2A(C634R) autophosphorylation and expression. Cells were treated with solvent (-) or 10 µM Cpd1 (+), for the indicated times. Whole cell lysates were prepared and subjected to SDS-PAGE and Western blotting with anti-pTyr antibody. After stripping, the filter was reblotted with anti-Ret antibody. Arrows indicate the partial and fully glycosilated forms of the Ret/MEN2A receptor. Following 2h of exposure to the drug, the receptor appeared partially dephosphorylated. At longer times, a complete tyrosine dephosphorylation was associated with reduced expression of the receptor
**Figure 2.** Effects of Cpd1 on human MTC cell lines harboring MEN2-associated RET mutants. A) Inhibition of Ret autophosphorylation. TT and MZ-CRC-1 cells, expressing respectively RET/MEN2A(C634W) and RET/MEN2B(M918T), were treated with the indicated concentrations of Cpd1, for 24 h. Control cells (C) received the solvent. Whole cell extracts were processed for Western blotting and probed with anti-pTyr and anti-Ret antibodies. As evidenced in anti-pTyr blots, cell treatment with the compound induced a dose-dependent inhibition of tyrosine phosphorylation of Ret receptors in both cell lines. A reduced expression of receptor concentrations was observed in cells treated with the highest concentrations of the drug. B) Antiproliferative effect. TT and MZ-CRC-1 cells were treated with increasing concentrations of Cpd 1 for 7 days and then counted with a Coulter Counter. Dose-response curves documented the ability of the drug to interfere with the proliferation potential of the two MTC cell lines.
**Figure 3**. Antitumor activity of Cpd 1 in nude mice harboring TT medullary thyroid carcinoma xenografts. Drug treatment started 25 days after s.c. inoculum of the tumor cells. Cpd 1 was delivered per os at 50 or 100 mg/Kg, twice in a day (2qd), for 10 consecutive days (indicated by arrows). Control mice received the vehicle. The treatment induced a significant dose-dependent inhibition of tumor growth. TWI were 60% (P < 0.005) and 80% (P< 0.0005) for the 50 mg/Kg and 100 mg/Kg doses, respectively.
**Figure 4**. Inhibition of Met autophosphorylation and expression in human papillary thyroid carcinoma cells (TPC-1) treated with Cpd1. A): TPC-1 cells were exposed to solvent (-) or the indicated concentrations of Cpd 1 for 72h. Equal amounts of protein were used for immunoprecipitation (IP) with anti-Met antibody or for the preparation of whole cell lysates (WCL). Immunoprecipitated proteins and WCLs were separated by SDS-PAGE, transferred to nitrocellulose membranes, and subjected to Western blotting with anti-pTyr or anti-Met antibodies. **B**): Cells were treated as in A. Cell extracts were immunoprecipitated with anti-pTyr antibody and probed with anti-Met antibody. C): Cells were serum-starved for 24h and exposed to solvent or Cpd1 (60 µM) during the last 18h. Then they were left untreated (-) or stimulated with 20ng/ml HGF (+), for 10 min. Cell lysates were immunoprecipitated with anti-Met and probed with anti-pTyr or anti-Met antibody. Drug treatment abolished the constitutive or HGF-induced Met tyrosine phosphorylation and induced a reduction of Met expression.
**Figure 5**. Inhibition of PDGF-R autophosphorylation and expression in whole cells by Cpd 1. A): 2N5A cells (NIH3T3 transformed by the COL1A1/PDGFB rearrangement generating autocrine stimulation of PDGF-R) were treated with Cpd 1 at the indicated concentrations, for 72 h. Cell extracts were immunoprecipitated with anti-PDGF-R and subjected to Western blotting with anti-pTyr or anti-PDGF-R antibodies. **B):** Swiss 3T3 cells were serum starved for 24 h and then treated with Cpd1 at the indicated concentrations, for 18 h. After stimulation with 1 nM PDGF for 5 min, whole cell lysates were prepared and subjected to Western blotting with anti-pTyr or anti-PDGF-R. Protein loading by anti-actin blot is shown. In both cell systems, a dose-dependent drug-induced inhibition of receptor tyrosine phosphorylation and expression was documented.
**Figure 6.** Inhibition of autophosphorylation and expression of FGF-R1 and FGF-R3 receptors in whole cells by Cpd1. **A):** Swiss3T3 cells were serum starved for 24 h and then treated with Cpd1 at the indicated concentrations, for 18 h. After stimulation with 100 ng/ml FGF for 5 min, whole cell lysates were prepared and subjected to Western blotting with anti-pTyr or anti-FGF-R1. Protein loading by anti-actin blot is shown. **B**): NIH3T3 transformed by the FGF-R3 mutant Y373C were treated with the indicated concentrations, for 72 h. Cell extracts were immunoprecipitated with anti-FGF-R3 and then subjected to Western blotting with anti p-Tyr or anti-FGF-R3. In both cell systems, a dose-dependent inhibition by Cpd 1 of the receptor tyrosine phosphorylation and expression was documented.
**Figure 7.** Effects of Cpd1 on cell lines expressing constitutively activated forms of Kit. A): Reversion of the transformed morphologic phenotype of NIH3T3 transfectants expressing exogenous mutated KIT (Δ559). Cells were treated with 20 µM Cpd1 and photographed 24h later under a phase-contrast microscope (original magnification X 100). **B**): Inhibition of Kit (Δ559) autophosphorylation and expression in NIH3T3 transfectants. Cells were treated with Cpd 1 at the indicated concentrations, for 72h. Cell lysates were immunoprecipitated with anti-Kit antibody and subjected to Western blotting with anti-p-Tyr or anti-Kit antibodies. **C**): Inhibition of expression and autocrine loop-activated autophosphorylation of c-Kit in the SCLC cell line N592. Cells were treated with Cpd 1 at the indicated concentrations, for 24h. Whole cell lysates were subjected to Western blotting with anti-Kit antibody or with an antibody specifically recognizing the tyrosine phosphorylated/activated Kit (p-Kit). In both cell systems, a dose-dependent drug-induced inhibition of receptor tyrosine phosphorylation and expression was documented.

### MATERIALS AND METHODS

### Cell lines and culture conditions

The following human medullary thyroid carcinoma (MTC) cell lines were used in this study. The TT cell line derived from a MEN2A-associated MTC characterized by expression of the RET oncogene carrying the mutation C634W. The MZ-CRC-1 cell line derived from MEN2B-associated MTC characterized by expression of the RET oncogene carrying the mutation M918T. TT cells were cultivated in Ham's F12 medium (BioWhittaker, Verviers, Belgium) supplemented with 15% fetal calf serum (FCS) (Life Technologies, Inc., Gaithersburg, MD) whereas MZ-CRC-1 cells were grown in Dulbecco's modified Eagle's medium (DMEM) (BioWhittaker) supplemented with 10% FCS. The human papillary thyroid carcinoma cell line TPC-1 which was used as a model of Met overexpression, was routinely cultivated in DMEM with 10% FCS. N592 cells, derived from a human SCLC, were characterized by Kit activation through autocrine stimulation by the SCF ligand. N592 cells were cultured in RPMI 1640 supplemented with 10% FCS. The mouse SWISS3T3 and NIH3T3 fibroblasts were cultured in DMEM with 10% calf serum (Colorado Serum Company, Denver, CO). In addition, NIH3T3 cells transfected with different oncogenes were used. NIH3T3^{MEN2A} transfectants express the short isoform of the RET-MEN2A (C634R) oncogene; NIH3T3^{KITΔ559} cells express KIT carrying the activating mutation Δ559 found in GISTs; NIH3T3^{FGFR3(Y373C)} express the FGF-R3 gene carrying the activating mutation Y373C found in a human multiple myeloma cell line. 2N5A cells are NIH3T3 cells transformed by the COL1A1/PDGFB rearrangement generating autocrine stimulation of PDGF-R. All NIH3T3 transfectants were maintained in DMEM plus 5% calf serum in a 10% CO₂ atmosphere.

### Antiproliferative assays

Cells were trypsinized after 3 days (NIH3T3 and NIH3T3^{MEN2A} cells) or 7 days (MTC cells) of treatment with Cpd1 and counted by a Coulter Counter (Coulter Electronics, Luton U.K.). The concentrations able to inhibit cell proliferation by 50% (IC₅₀) were calculated from the dose-response curves. Each experiment was performed in duplicate

### Antibodies

The following polyclonal antibodies were used: anti-Ret recognizing a COOH-terminal sequence (aa 1000-1014) common to the two Ret isoforms (Borrello M. G., et al., Mol. Cell Biol. 16, 2151, 1996); anti-cKit, anti-Met and anti-FGF-R3 from Santa Cruz Biotechnology (Santa Cruz, CA); anti-actin from Sigma (St. Louis, MO); anti-PDGF-R α/β from Upstate Biotechnology (Lake Placid, NY); anti phospho-cKit (Tyr 719) from Cell Signaling Technology (Beverly, MA). The mouse monoclonal anti-phosphotyrosine (anti-pTyr) 4G10 and anti-FGF-R1 antibodies were from Upstate Biotechnology.

### Immunoprecipitation and Western blotting

For whole-cell extract preparation, cells were lysed in sodium dodecyl sulfate (SDS) sample buffer (62.5 mM Tris-HCl (pH 6.8), 2% SDS) with 1 mM PMSF, 10 µg/ml pepstatin, 12.5 µg/ml leupeptin, 100 KIU aprotinin, 1 mM sodium orthovanadate, 1mM sodium molybdate. Protein concentration was determined in appropriately diluted aliquots by the bicinchionic acid (BCA) method (Pierce, Rockford, IL), then samples were adjusted to a final concentration of 10% glycerol, 5% β-mercaptoethanol, 0.001% bromophenol blue.

For immunoprecipitation experiments, cells were treated with Cpd1 or solvent for the indicated times. Cell monolayers were rinsed twice with cold phosphate-buffered saline plus 0.1 mM sodium orthovanadate and then left for 20 min on ice in lysis buffer (50mM HEPES pH7.6, 150mM NaCl, 10 % glycerol, 1% Triton X-100, 1.5 mM MgCl₂, 1 mM EGTA, 100 mM NaF, 10 mM sodium pyrophosphate, 10 µg/ml antipain, 20 µg/ml chymostatin, 10 µg/ml E64, 1 mg/ml pefabloc SC). Cells were then collected, aspirated through a 22-gauge needle and centrifuged at 10000g, for 20 min, at 4 °C. Protein concentration was determined by the BCA reagent (Pierce). Cell extracts were incubated with protein A-agarose and the indicated antibody for 2 hr at 4°C under rotation. After washing 3 times with 20mM HEPES pH 7.6, 150 mM NaCl, 10% glycerol, 0.1% Triton X-100, the immunoprecipitates were eluted with complete sample buffer.

Normalized immunoprecipitates (0.5 - 4 mg of cell extracts) or whole-cell lysates (30 - 60 µg) were resolved on SDS-PAGE and transferred to nitrocellulose filters. Membranes were incubated with primary antibodies at 4°C, overnight. Immunoreactive bands were revealed by horseradish peroxidase-coniugated anti-mouse or anti-rabbit antibodies using enhanced chemiluminescence detection systems from Amersham Biosciences (Little Chalfont, United Kingdom) or Pierce.

### In vivo studies

All experiments were carried out using female athymic nude CD-1 mice, 8-11-weeks old (Charles River, Calco, Italy). Mice were maintained in laminar flow rooms with constant temperature and humidity. Experimental protocols were approved by the Ethic Committee for Animal Experimentation of the Istituto Nazionale per lo Studio e la Cura dei Tumori (Milan, Italy), according to the United Kingdom Coordinating Committee on Cancer Research Guidelines (Workman P. et al., British Journal of Cancer, 77, 1, 1998).

The MTC cells TT (1.6x10⁷ cells) were s.c. inoculated in mice as a cell suspension from in vitro cell culture. Each control or drug-treated group included 8-10 tumors. Tumor cells were injected on day 0, and tumor growth was followed by biweekly measurements of tumor diameters with a Vernier caliper. Tumor weight (TW) was calculated according to the formula: TW (mg) = tumor volume (mm³) = d²xD/2, where d and D are the shortest and the longest diameter, respectively. Drug treatment started when tumors were just measurable (mean TW about 50 mg), 25 days after the inoculum of tumor cells. Cpd1 was dissolved in 5% ethanol, 5% Cremophor EL, 90% saline (0.9% NaCl) and delivered per os twice in a day (2qd) by a daily schedule for 10 days. Control mice received the solvent solution.

Drug efficacy was assessed as percentage TW inhibition (TWI% in drug-treated versus control mice expressed as: TWI%=100 - (mean TW treated/mean TW control x 100). For statistical analysis, TW in control and treated mice were compared on the day of TWI% evaluation, by Student's t test (two-tailed). P values less than 0.05 were considered statistically significant.

Preparation of (E) 1,3-dihydro-4-hydroxybenzyliden-5,6-dimethoxy-(1H)-indol -2-one (Cpd 1).

### 1) Synthesis of 2-nitro-4,5-dimethoxyphenylacetic acid

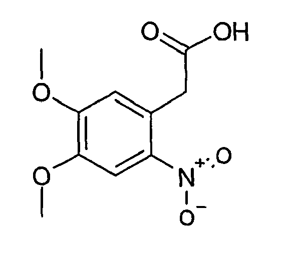

C₁₀H₁₁NO₆, M.W. 241,20

45 g (0.23 moles, 1 eq.) of 3,4-dimethoxyphenylacetic acid were dissolved in 100mL (2.2 volumes) glacial acetic acid at 28°C-35°C, under N₂ atmosphere and mechanic stirring. The solution was cooled to 15°-20°C and added with a mixture of fuming nitric acid (98%, 33 mL) in glacial acetic acid (25 mL) over a period of 45'. Once the addition was completed, precipitation of a red solid was observed. The suspension was poured in ice water (600 mL) and kept under stirring for 2h. The solid was filtrated, washed with water and dried at 60°C for 8h. 44g of the end product were obtained.
Yield 79.3% (mmol/mmol)
TLC (SiO₂; Ethyl acetate 10/AcOH 0.5) Rf_{acid} = 0.6; Rf_{product} = 0.5
m.p. 199°-202°C
1H-NMR, (DMSO): 3.9 ppm (s, 6H); 4.0 ppm (s., 2H); 7.12 ppm (s., 1H); 7.7 ppm (s., 1H)

### 2) Synthesis of 1,3-dihydro-5,6-dimethoxy-(1H)-indol-2-one

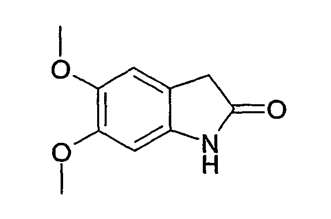

C₁₀H₁₁NO₃, M.W., 193,11

9.2 g (38.14 mmoles, 1 eq.) of 3,4-dimethoxy-2-nitro-phenylacetic acid were suspended in glacial acetic acid (92 mL, 10 volumes) at 25°C under N₂ atmosphere and mechanic stirring. The suspension was added with Fe° powder, 325 mesh, 97% (12.0g, 214.86 mmoles, 5.6 eq.) in two equal portions. The first portion was added at room temperature; then the mixture was refluxed and 30 min later the second Fe° portion was added. 30' later the reaction was complete, TLC (SiO₂; CHCl₃ 9/MeOH 1), Rfₙᵢₜᵣₒ = 0.65, Rf_{indolinone} = 0.71.

The grey suspension was cooled to room temperature, the acetic acid was evaporated under low pressure to a crude solid, which was suspended in chloroform (200 mL). The salts were filtrated off and the organic phase was washed with a NaCl saturated solution (100 mL), dried over Na₂SO₄ and evaporated to dryness. The solid was suspended in ethyl ether (35 mL) for 30', filtrated and dried at 50°C for 2h. 6.7 g of a beige solid were obtained.
Yield 90.9% (mmol/mmol)
m.p. 199°-201°C
TLC (SiO₂; Ethyl acetate 10/AcOH 0.5) Rf_{acid} = 0.6; Rf_{product} = 0.5
1H-NMR, (DMSO): 3.4 ppm (s, 2H); 3.69 ppm (s., 3H); 3.72 ppm (s., 3H); 6.49 ppm (s., 1H); 6.92 ppm (s., 1H); 10.15 (S., 1H).

### 3) Synthesis of (E)-1,3-dihydro-4-hydroxybenzyliden-5,6-dimethoxy-1H-indol-2-one

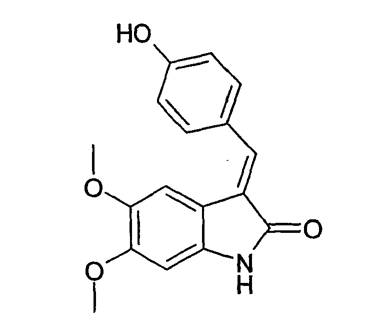

C₁₇H₁₅NO₄, M.W. 297,31

6.7 g (36.9 mmoles, 1 eq.) of 1,3-dihydro-5,6-dimethoxy-(1H)-indol-2-one were dissolved in anhydrous DMSO (50 mL) at room temperature. The solution was added with 4-hydroxybenzaldehyde (5.41 g, 44.3 mmoles, 1.2 eq.) and piperidine (4.38 g, 44.3 mmoles, 1.2 eq.), then stirred for 16 hours. The mixture was poured in H₂O (250 mL) and HCl 0.5N (150mL), and precipitation of a solid was observed. The solution was cooled to 5°-10°C for 1 h, filtrated and dried under vacuum at 80°C for 2h. 13g of wet solid were obtained and then crystallized from absolute ethanol, yielding 6.77g of end product.
Yield 61.6% (mmol/mmol)
m.p. 238°-240°C
Rf (Silica; Ethyl acetate 100%) = 0.68
¹H-NMR, (DMSO): 3.6 ppm (s, 3H); 3.8 ppm (s., 3H); 6.5 ppm (s., 1H); 6.9 ppm (d., 2H, J=8.6 Hz); 10 ppm (broad s.); 12.8 ppm (s.).

The E configuration of the esocyclic double bond in position 2 was determined by means of 1D NOE NMR experiments.

## Claims

1. The use of the compound (E)-1,3-dihydro-5,6-dimethoxy-3-[(4-hydroxyphenyl)methylene]-2H-indol-2-one or of non-toxic salts or isomers thereof for the preparation of a medicament for the treatment of tumors involving a tyrosine kinase selected from Met, PDGF-R, FGF-R1, FGF-R3, Kit or RET oncoproteins including MEN2 associated mutations.

2. The use according to claim 1, wherein the activated sequence mutations are RET/MEN2A (C634R), RET/MEN2A (C634W) and RET/MEN2B (M918T).

3. The use according to claim 1-3, for the treatment of medullary thyroid carcinomas, pheochromocytoma, parathyroid hyperplasia, enteric ganglioneuroma.

4. The use according to claim 1, for the treatment of tumors bearing a Met-activating alteration.

5. The use according to claim 4, wherein said tumors are of epithelial origin.

6. The use according to claim 5, for the treatment of kidney tumor.

7. The use according to claim 1, for the treatment of tumors expressing constitutively-activated Kit.

8. The use according to claim 7, wherein Kit is constitutively activated following to sequence mutations or involvement in autocrine loops.

9. The use according to claim 7, for the treatment of gastrointestinal stromal tumors, small cell lung carcinomas, leukemias or seminomas.

10. The use according to claim 1, for the treatment of tumors involving the uncontrolled activation of PDGF-R.

11. The use according to claim 10, wherein said tumors are gliomas and dermatofibrosarcoma protuberans.

12. The use according to claim 1, for the treatment of tumors highly expressing FGF-R1 and/or its ligand bFGF.

13. The use according to claim 12, wherein said tumors are melanomas and gliomas.

14. The use according to claim 1, for the treatment of tumors expressing constitutive activating forms of FGF-R3.

15. The use according to claim 14, wherein said tumors are multiple myeloma, bladder and cervix carcinomas.

16. The use according to claims 10 and 12, for the inhibition of tumor angiogenesis.

17. A pharmaceutical composition containing as active ingredient the compound (E)-1,3-dihydro-5,6-dimethoxy-3-[(4-hydroxyphenyl)methylene]-2H-indol-2-one or a pharmaceutically acceptable salt thereof in combination with a pharmaceutically acceptable carrier, excipient or diluent.

18. The pharmaceutical composition according to claim 17, wherein said pharmaceutically acceptable carrier or diluent is suitable for oral or parenteral administration.

19. The pharmaceutical composition according to claim 17, further I comprising a anti-tumor or anti-cancer agent which is different from (E)-1,3-dihydro-5,6-dimethoxy-3-[(4-hydroxyphenyl)methylene]-2H-indol-2-one.

20. The pharmaceutical composition according to claim 19, wherein said anti-tumor or anti-cancer agent is selected from the group consisting of adriamycin, daunomycin, methotrexate, vincristin, 6-mercaptopurine, cytosine arabinoside, cyclophosphamide, 5-FU, hexamethylmelamine, carboplatin, cisplatin, idarubycin, paclitaxel, docitaxel, topotecan, irinotecam, gencitabine, Lpam, BCNU and VP-16.

## Patentansprüche

1. Verwendung der Verbindung (E)-1,3-Dihydro-5,6-dimethoxy-3-[(4-hydroxyphenyl)methylen]-2H-indol-2-on oder eines nichttoxischen Salzes oder Isomers davon zur Herstellung eines Medikaments zur Behandlung von Tumoren, die zusammenhängen mit einer Tyrosinkinase, ausgewählt aus Met, PDGF-R, FGF-R1, FGF-R3, Kit oder RET-Oncoproteinen, einschließlich MEN2-assoziierte Mutationen.

2. Verwendung nach Anspruch 1, wobei die Mutationen der aktivierten Sequenz RET/MEN2A (C634R), RET/MEN2A (C634W) und RET/MEN2B (M918T) sind.

3. Verwendung nach Anspruch 1 bis 3 zur Behandlung von medullären Schilddrüsenkarzinomen, Phäochromozytomen, Nebenschilddrüsenhyperplasien, enterischen Ganglioneuromen.

4. Verwendung nach Anspruch 1 zur Behandlung von Tumoren, die eine Met-aktivierende Veränderung tragen.

5. Verwendung nach Anspruch 4, wobei die Tumore von epitheliärem Ursprung sind.

6. Verwendung nach Anspruch 5 zur Behandlung von Nierentumoren.

7. Verwendung nach Anspruch 1 zur Behandlung von Tumoren, die konstitutiv aktiviertes Kit exprimieren.

8. Verwendung nach Anspruch 7, wobei das Kit konstitutiv aktiviert ist nach Sequenzmutationen oder Beteiligung an "Autocrine Loops".

9. Verwendung nach Anspruch 7 zur Behandlung von gastrointestinalen Bindegewebstumoren, kleinzelligen Lungenkarzinomen, Leukämien oder Seminomen.

10. Verwendung nach Anspruch 1 zur Behandlung von Tumoren, bei denen die unkontrollierte Aktivierung von PDGF-R auftritt.

11. Verwendung nach Anspruch 10, wobei die Tumore Gliome und Dermatofibrosarcoma protuberans sind.

12. Verwendung nach Anspruch 1 zur Behandlung von Tumoren, die FGF-R1 und/oder dessen Ligand bFGF stark exprimieren.

13. Verwendung nach Anspruch 12, wobei die Tumore Melanome und Gliome sind.

14. Verwendung nach Anspruch 1 zur Behandlung von Tumoren, die konstitutiv aktivierende Formen von FGF-R3 exprimieren.

15. Verwendung nach Anspruch 14, wobei die Tumore multiple Myelome, Blasen und Gebärmutterhalskarzinome sind.

16. Verwendung nach Anspruch 10 und 12 zur Inhibierung der Tumorangiogenese.

17. Pharmazeutische Zusammensetzung, enthaltend als aktiven Bestandteil die Verbindung (E)-1,3-Dihydro-5,6-dimethoxy-3-[(4-hydroxyphenyl)methylen]-2H-indol-2-on oder ein pharmazeutisch annehmbares Salz davon in Kombination mit einem pharmazeutisch annehmbaren Träger, Hilfsstoff oder Verdünnungsmittel.

18. Pharmazeutische Zusammensetzung nach Anspruch 17, wobei der pharmazeutisch annehmbare Träger oder das Verdünnungsmittel zur oralen oder parenteralen Verabreichung geeignet ist.

19. Pharmazeutische Zusammensetzung nach Anspruch 17, des Weiteren umfassend ein Antitumor- oder Antikrebsmittel, das von (E)-1,3-Dihydro-5,6-dimethoxy-3-[(4-hydroxyphenyl)methylen]-2H-indol-2-on verschieden ist.

20. Pharmazeutische Zusammensetzung nach Anspruch 19, wobei das Antitumor- oder Antikrebsmittel ausgewählt ist aus der Gruppe, bestehend aus Adriamycin, Daunomycin, Methotrexat, Vincristin, 6-Mercaptopurin, Cytosinarabinosid, Cyclophosphamid, 5-FU, Hexamethylmelamin, Carboplatin, Cisplatin, Idarubycin, Paclitaxel, Docitaxel, Topotecan, Irinotecan, Gencitabin, Lpam, BCNU und VP-16.

## Revendications

1. Utilisation du composé (E)-1,3-dihydro-5,6-diméthoxy-3-[(4-hydroxyphényl)méthylène]-2H-indol-2-one ou de sels ou isomères non toxiques de celui-ci pour la préparation d'un médicament pour le traitement de tumeurs impliquant une tyrosine kinase choisie parmi les oncoprotéines Met, PDGF-R, FGF-R1, FGF-R3, Kit ou RET incluant les mutations associées MEN2.

2. Utilisation selon la revendication 1, dans laquelle les mutations de séquence activées sont RET/MEN2A (C634R), RET/MEN2A (C634W) et RET/MEN2B (M918T).

3. Utilisation selon la revendication 1-3, pour le traitement du carcinome médullaire de la thyroïde, du phéochromocytome, de l'hyperplasie parathyroïdienne, du ganglioneurome entérique.

4. Utilisation selon la revendication 1, pour le traitement de tumeurs portant une altération activant Met.

5. Utilisation selon la revendication 4, dans laquelle lesdites tumeurs sont d'origine épithéliale.

6. Utilisation selon la revendication 5, pour le traitement de tumeur du rein.

7. Utilisation selon la revendication 1, pour le traitement de tumeurs exprimant Kit activé de manière constitutive.

8. Utilisation selon la revendication 7, dans laquelle Kit est activé de façon constitutive suite à des mutations de séquence ou à une implication dans des boucles autocrines.

9. Utilisation selon la revendication 7, pour le traitement de tumeurs stromales gastro-intestinales, de carcinomes du poumon à petites cellules, de leucémies ou de séminomes.

10. Utilisation selon la revendication 1, pour le traitement de tumeurs impliquant l'activation non contrôlée du PDGF-R.

11. Utilisation selon la revendication 10, dans laquelle lesdites tumeurs sont les gliomes et le dermatofibrosarcome de Darier et Ferrand.

12. Utilisation selon la revendication 1, pour le traitement de tumeurs exprimant fortement FGF-R1 et/ou son ligand bFGF.

13. Utilisation selon la revendication 12, dans laquelle lesdites tumeurs sont des mélanomes et des gliomes.

14. Utilisation selon la revendication 1, pour le traitement de tumeurs exprimant des formes activatrices constitutives de FGF-R3.

15. Utilisation selon la revendication 14, dans laquelle lesdites tumeurs sont le myélome multiple, les carcinomes de la vessie et du col.

16. Utilisation selon les revendications 10 et 12, pour l'inhibition de l'angiogénèse de tumeur.

17. Composition pharmaceutique contenant en tant qu'ingrédient actif le composé (E)-1,3-dihydro-5,6-diméthoxy-3-[(4-hydroxyphényl)méthylène]-2H-indol-2-one ou un sel acceptable sur le plan pharmaceutique de celui-ci en combinaison avec un support, excipient ou diluant acceptable sur le plan pharmaceutique.

18. Composition pharmaceutique selon la revendication 17, dans laquelle ledit support ou diluant acceptable sur le plan pharmaceutique est adapté à une administration orale ou parentérale.

19. Composition pharmaceutique selon la revendication 17, comprenant en outre un agent anti-tumoral ou anti-cancéreux qui est différent du (E)-1,3-dihydro-5,6-diméthoxy-3-[(4-hydroxyphényl)méthylène]-2H-indol-2-one.

20. Composition pharmaceutique selon la revendication 19, dans laquelle ledit agent anti-tumoral ou anti-cancéreux est choisi dans le groupe constitué de l'adriamycine, la daunomycine, le methotrexate, la vincristine, la 6-mercaptopurine, la cytosine arabinoside, le cyclophosphamide, le 5-FU, l'hexaméthylmélamine, le carboplatine, le cisplatine, l'idarubycine, la paclitaxel, le docitaxel, le topotecan, l'irinotecam, la gencitabine, le Lpam, le BCNU et le VP-16.
